# FASCICULE DE BREVET EUROPEEN

(11) **EP 0 383 676 B1**
(45) Date de publication et mention de la délivrance du brevet: **21.07.1993**
(21) Numéro de dépôt: 90400392.8
(22) Date de dépôt: 13.02.1990
(51) Int. Cl.: A61F 2/24

(54) **Valve cardiaque prothétique**
Herzklappenprothese
Prosthetic heart valve

(30) Priorité: 15.02.1989 FR 8901945
(43) Date de publication de la demande: 22.08.1990
(73) Titulaire: DASSAULT AVIATION, F-92420 Vaucresson (FR); Lapeyre, Didier, Dr., F-27120 Pacy sur Eure (FR)
(72) Inventeur: Perrier, Philippe, F-78860 St. Nom La Bretèche (FR); Lapeyre, Didier, F-27120 Pacy sur Eure (FR)
(74) Mandataire: Orès, Bernard

(56) Documents cités:
- EP-A- 0 133 608
- EP-A- 0 207 594
- EP-A- 0 289 404
- FR-A- 2 587 614
- US-A- 4 078 268

## Description

La présente invention est relative à une valve cardiaque prothétique mécanique destinée à être implantée chez l'homme pour le remplacement d'une valve cardiaque naturelle détériorée par un processus pathologique infectieux ou dégénératif. On connaît les critères de sélection auxquels doit maintenant répondre une valve cardiaque prothétique. Ces critères ont été fixés par la F.D.A. (Food and Drug Administration) qui est l'organisme américain de règlementation dans ce domaine.

Il s'agit des sept critères suivants :
1) Le premier critère est défini par la **compatibilité avec le sang** des matériaux utilisés : ces matériaux doivent être compatibles avec l'organisme et se comporter de façon neutre vis-à-vis des processus naturels de coagulation, c'est-à-dire ne pas induire à leur contact des phénomènes de thrombose. La thrombose locale provoque en effet des embolies et peut même gêner le fonctionnement de la valve. Plusieurs matériaux se comportent de façon satisfaisante à ce point de vue : alliages métalliques spécifiques, tels que Chrome, Nickel-Tungstène, Titane, et surtout carbone pyrolitique, matériau le plus souvent utilisé aujourd'hui dans les prothèses valvulaires disponibles dans le commerce.
2) Le deuxième critère est défini par **la qualité du sillage** induit dans le courant sanguin par le dispositif prothétique. Les zones de turbulences, de recirculation et de vitesses nulles entraînent, en effet, les mêmes phénomènes de thromboses et d'embolies, à tel point qu'on a pu dire que les perturbations générées dans l'écoulement du sang étaient plus thrombogènes que le matériau utilisé. C'est la raison pour laquelle les valves biologiques ("bioprothèses"), qui reproduisent la forme de l'écoulement naturel, ont un grand avantage sur les prothèses mécaniques. On sait, en effet, que ces valves ne nécessitent pas la prise de médicaments anticoagulants. Ces deux facteurs thrombogènes (Matériau et Sillage) sont aujourd'hui encore imparfaitement maîtrisés sur les prothèses valvulaires mécaniques, ce qui oblige à mettre les malades sous anti-coagulants à vie et constitue donc un inconvénient majeur par rapport aux bioprothèses.
   Malheureusement les valves biologiques ne sont pas pour autant la solution idéale, car l'expérience clinique montre qu'elles ne présentent pas, contrairement aux valves mécaniques, une durée de vie suffisante. Le chirurgien, en effet, est souvent amené, du fait de leur détérioration progressive, à les remplacer au prix d'une réintervention lourde. Il n'a pas été possible jusqu'à maintenant de reproduire avec une prothèse mécanique les caractéristiques d'écoulement d'une valve naturelle. C'est précisément l'objectif de la présente invention.
3) Le troisième critère pour les valves mécaniques est, en effet, la **durabilité** : ces valves doivent faire la preuve, par des tests de fatigue accélérée, d'une durée de vie équivalente à 560 millions de cycles, soit l'équivalent d'environ 15 années dans les conditions de fonctionnement physiologiques (fréquence moyenne de 70 battements cardiaques par minute). Cette durée de vie mécanique est en rapport avec le dessin géométrique de la valve, avec les moyens de guidage et de butée adoptés pour le ou les clapets qui la constituent et avec les caractéristiques mécaniques du matériau utilisé.
4) Le quatrième critère est directement lié au deuxième (sillage) et concerne **les pertes de charges** que provoque la prothèse mécanique sur l'éjection systolique ou le remplissage diastolique du ventricule. Une partie de ces pertes de charge est inévitable, car inhérente à la diminution de la surface active -"EOA, Effective Orifice Area"- (cette diminution de surface active est liée à la réduction de diamètre provoquée, d'une part, par l'anneau de textile indispensable pour la mise en place chirurgicale de la valve et, d'autre part, par l'épaisseur de la bague métallique qui supporte le dispositif du ou des clapets et leurs moyens d'articulation entre les positions d'ouverture et de fermeture). Une deuxième partie de ces pertes de charges revient à la disposition géométrique des clapets et, principalement, à leur orientation par rapport au courant sanguin.
   Sur un orifice anatomique petit, et notamment chez l'enfant, ce facteur est très important. Il est ainsi couramment observé, avec les tailles 19 et 21 mm, des gradients de pressions supérieurs à 40 mm de Hg entre le ventricule gauche et l'aorte, ce qui implique un travail supplémentaire pour le muscle cardiaque. Ce gradient de pression peut atteindre des chiffres supérieurs à l'effort, lorsque le débit cardiaque augmente. Le chirurgien utilisera donc plus volontiers les valves mécaniques qui, pour une taille donnée, sont les moins sténosantes.
5) Le cinquième critère retenu concerne le **temps de fermeture** des clapets lorsque le ventricule se contracte ou se relâche. Il est lié à la définition géométrique de la valve et de son ou ses clapets et conditionne le t**aux de régurgitation** qui représente actuellement, dans le meilleur des cas, une fraction au moins égale à 5 % du volume sanguin mobilisé au cours de chaque cycle. Lorsqu'un malade est porteur de deux valves sur le même ventricule, la fuite est donc au minimum de l'ordre de 10 %, ce qui constitue une fatigue non négligeable pour le muscle cardiaque.
6) Le sixième critère est la **fuite statique**, à savoir l'étanchéité mécanique de la valve prothétique lors de sa fermeture. Pour les mêmes raisons, ce critère influe également sur le rendement hydraulique de la valve.
7) Le septième critère peut être considéré comme la somme des six autres ; il concerne **le bon fonctionnement de la valve sur l'homme**, observé sur plusieurs années, avec le minimum possible d'accidents thrombo-emboliques. Quelques valves mécaniques donnent des résultats tout à fait acceptables. Néanmoins, la morbidité et la mortalité restent significatives, puisque le nombre d'accidents emboliques se situent autour de 2 % par an et par malade et que la mortalité dans un grand nombre de cas est d'origine cardiaque, donc directement ou indirectement liée à la prothèse valvulaire.

Il faut noter, à ce sujet, que ces phénomènes pathologiques sont plus fréquents sur les malades porteurs d'une valve en position mitrale, car le régime d'écoulement du sang à travers la valve, dans cette configuration, -**à pression basse et pendant un temps plus long**-, favorise la thrombose dans les oreillettes, ceci d'autant plus que le dispositif prothétique est plus sténosant, plus régurgitant, plus turbulent et moins étanche.

En configuration mitrale aucune prothèse valvulaire, quelle soit mécanique ou biologique, ne donne pleinement satisfaction. C'est la raison pour laquelle il importe d'apporter des améliorations aux produits existants.

Il est intéressant de rappeler que les premières valves prothétiques proposées au début de la chirurgie cardiaque, dans le début des années 60, furent des valves qui tentaient de reproduire la forme des valves cardiaques naturelles. Du fait de leur complexité et des possibilités technologiques disponibles à cette époque, ces prothèses n'étaient pas fiables et il fallut les abandonner. Ce fut une révolution quand, précisément pour résoudre ce problème, Starr et Edwards proposèrent de s'écarter radicalement du modèle naturel en inventant la valve à bille maintenue dans une cage rigide. La fonction de valve était ainsi reproduite d'une façon sûre, bien que grossière. Cette valve est encore utilisée de nos jours par un certain nombre de chirurgiens.

A l'évidence, la présence d'une bille dans le courant sanguin entraîne une importante perte de charge et des perturbations considérables sur l'écoulement. Les observations faites sur l'homme ont bien confirmé cet inconvénient. C'est la raison pour laquelle Bjork proposa, quelques années plus tard, de remplacer la bille par un disque circulaire pivotant sur un dispositif articulaire. Les pertes de charges sont ainsi réduites. Cependant, pour des raisons mécaniques, il n'est pas possible de réaliser un basculement complet à 90° du disque pivotant. L'angle d'ouverture est de l'ordre de 60° à 75°. Il existe donc un grand et un petit orifice. Les conséquences sur le sillage induites par ce dispositif asymétrique sont facilement imaginables : il existe une importante zone de turbulence, en aval de la valve, au dessus du petit orifice, responsable de phénomènes thrombemboliques et, parfois, de dysfonctionnement du clapet. Ces anomalies sont bien mises en évidence aujourd'hui par les techniques modernes de visualisation d'écoulements (Doppler-Anémométrie).

Une troisième génération de valve, à partir de la fin des années 70, a apporté une amélioration significative sur ce point en utilisant, non pas un disque basculant, mais deux hemi-disques pivotant sur des micro-articulations proches du diamètre. L'angle d'ouverture des deux clapets se rapproche de la perpendiculaire. Le sillage généré est plus symétrique. Les pertes de charges sont sensiblement moindre. Les valves de ce type sont aujourd'hui le plus couramment utilisées en clinique humaine.

En résumant, un disque est meilleur qu'une bille ; deux hemi-disques (valves "papillon") sont plus performants qu'un seul disque. Cependant, dans les valves de ce type les axes d'articulations des clapets sont situés en position centrale, dans la zone des vitesses rapides et, comme l'ouverture n'est jamais à 90° (car il en résulterait une trop importante fraction de régurgitation), des zones de turbulences thrombogènes subsistent avec un sillage imparfait.

On connaît le Brevet US-4 276 558, correspondant au Brevet FR-2 407 709, qui décrit une prothèse de valve cardiaque du type comprenant un corps pourvu d'un passage central d'écoulement du sang, des moyens de réglage de cet écoulement du sang (clapets) pivotant sous l'action de l'écoulement sanguin, entre une position de fermeture et une position d'ouverture, et des moyens d'articulation de ces moyens de réglage.

La valve, objet de ces deux Brevets, comporte des moyens d'articulation comprenant : des cavités ménagées dans le corps annulaire de la valve, qui présentent une surface de portée en forme de surface de révolution, et des clapets pourvus de saillies destinées à pénétrer dans les cavités précitées et à balayer les surfaces de révolution avec leurs extrémités, qui sont chacune en contact avec une des surfaces de portée.

De façon plus précise, ces cavités sont ménagées dans les faces -internes et plates- de deux oreilles parallèles et diamétralement opposées, faisant saillie axialement vers l'amont à partir de la périphérie du corps annulaire de la valve prothétique.

Bien que la valve, objet des Brevets américain et français précités, soit acceptable au point de vue des critères de sélection évoqués plus haut, il y a toutefois lieu de remarquer que, en ce qui concerne la thromborésistance, celle-ci ne peut pas atteindre les valeurs optimales à cause de la présence de zones de stagnation, au moins partielle, du sang qui sont constituées par les cavités dans lesquelles tournent les saillies des clapets ; en outre, étant donné que ces clapets ne présentent qu'un seul degré de liberté, défini par la rotation autour d'un axe passant par les deux cavités opposées, ménagées dans les oreilles précitées, il est inévitable que le balayage des cavités se fasse avec préjudice des globules rouges, qui sont écrasés dans cette cavité ; de plus, les caractéristiques dynamiques de l'écoulement sanguin à travers cette valve sont altérées par la disposition centrale des clapets en position ouverte dans la zone des vitesses rapides.

Les Demandeurs ont déjà proposé une valve cardiaque prothétique qui présente un comportement excellent, notamment du point de vue de la thromborésistance et de l'hémodynamique, -qui répond donc aux deux critères de sélection principaux d'une valve prothétique, soulignés plus haut-, tout en étant très satisfaisante du point de vue des autres paramètres définissant les critères de sélection précités. Cette valve fait l'objet du Brevet FR-2 587 614 et du Brevet correspondant US-A - 4 820 299. En particulier, il est facile de vérifier que, dans la valve objet de ces deux derniers Brevets, on tient compte de l'influence de la disposition des clapets d'obturation en position ouverte sur la turbulence ainsi que sur le temps de fermeture (à savoir sur la rapidité de fermeture des clapets), lors de l'inversion du sens de l'écoulement sanguin. Cette valve tient également compte de la configuration anatomique des valves cardiaques naturelles, lesquelles présentent un élargissement de section vers l'aval, correspondant à :
- l'entrée dans le ventricule, en position mitrale, et
- aux trois sinus, en position aortique.

De façon plus précise, cette valve tient compte du fait que, près de sa paroi, l'écoulement sanguin est dévié (diverge) vers la périphérie de la valve en débit direct et converge vers le centre de la valve en débit inverse.

En outre, la disposition des clapets au voisinage des parois de la valve et le fait que l'extrémité aval des clapets se referme vers le centre de la valve présentent, à l'évidence, plusieurs avantages :
- dégagement complet de la partie centrale de la valve, où l'écoulement est le plus fort ;
- possibilité de placer les clapets dans une position parallèle à l'axe de la valve, profitant ainsi de la déviation (convergence) d'origine anatomique de l'écoulement sanguin en débit inverse, telle qu'évoquée plus haut, pour amorcer la fermeture ;
- mouvement des clapets balayant sensiblement les mêmes zones que la valve naturelle remplacée et ne risquant donc pas de rencontrer d'obstacles au fonctionnement ; en particulier, dans le cas de clapets dont le bord de fuite s'ouvre de la périphérie vers le centre, un repli éventuel des tissus au voisinage de la base peut empêcher complètement l'ouverture, alors que dans le cas de la présente invention ce repli ne peut que limiter légèrement l'ouverture complète.

Bien entendu, dans le cas de remplacement d'une valve aortique, il sera préférable de disposer d'une valve prothétique avec les trois clapets en face des trois sinus de la valve aortique naturelle ainsi remplacée. La disposition de l'axe d'articulation des clapets le plus prêt possible de la paroi et du bord inférieur de ces clapets, en position ouverte, permet une fermeture rapide de ces clapets. De plus, cette valve comporte des moyens appropriés pour minimiser l'usure des moyens de guidage en rotation et de retenue des clapets, ce qui a une incidence sur la longévité et la fiabilité d'une valve cardiaque prothétique. Ces moyens de minimisation de l'usure des moyens de guidage en rotation et de retenue des clapets sont les suivants :
- écartement maximal des points de butée qui limitent le mouvement des clapets à l'ouverture ou à la fermeture, de façon à obtenir les mouvements nécessaires à la limitation du mouvement avec les efforts les plus faibles possibles,
- augmentation des surfaces des clapets qui viennent en contact avec la base de la valve dans les positions extrêmes, de façon à obtenir les efforts nécessaires avec les pressions les plus faibles possibles (cette situation contribue également à diminuer le bruit du fonctionnement),
- élimination de zones ponctuelles et fixes de frottement des clapets sur la base de la valve prothétique au cours du mouvement.

Il est facile en outre de vérifier que la structure de cette valve prothétique est telle que toutes ses parties sont accessibles à l'écoulement sanguin au cours du cycle de fonctionnement, ce qui évite la formation de zones de stagnation du sang favorisant la formation de caillots. A cet effet, cette valve apporte un soin tout à fait particulier au niveau des articulations des clapets, alors que le problème des zones de stagnation est très mal résolu dans la plupart des valves prothétiques existant actuellement, en raison de la configuration adoptée pour les moyens d'articulation des clapets.

Bien que la valve objet des deux Brevets FR-2 587 614 et US-SN-06/910 621, rappelés plus haut, répond de façon excellente aux critères de sélection susdits, ainsi qu'aux différentes dispositions déjà rappelées, la présente invention vise à réaliser une valve cardiaque prothétique qui, tout en satisfaisant aux dispositions de la première valve, comporte des moyens de guidage en rotation et de retenue des clapets qui, tout en évitant la formation de zones de stagnation de l'écoulement sanguin, présente en outre une configuration qui se prête au revêtement du corps de la valve, ainsi que de ses moyens de guidage en rotation et de retenue, par du carbone pyrolytique, qui est un matériau thromborésistant et résistant à l'usure.

La présente invention à pour objet une valve cardiaque prothétique comprenant une base et au moins deux clapets, chaque clapet comprenant :
. un bord d'attaque qui, en position de fermeture, est au contact de la paroi interne de la base et en position d'ouverture se situe du côté amont de l'écoulement,
. un bord de fuite qui, en position de fermeture, est au contact du ou des bords de fuite du ou des autres clapets et, en position d'ouverture, se situe du côté aval de l'écoulement,
. deux bords latéraux constituant des profils de transition entre le bord d'attaque et le bord de fuite de chaque clapet, chaque bord latéral délimitant, latéralement vers l'extérieur, une zone de transition qui correspond à un coin du clapet et comprenant un segment amont, appartenant au bord d'attaque, et un segment aval appartenant au bord de fuite,

la ligne définie par la succession du bord d'attaque, du bord de fuite et des bords latéraux de chaque clapet constituant le contour de ce clapet et délimitant deux faces opposées, dont :
. une face interne qui, en position d'ouverture, est sensiblement parallèle à l'axe de l'écoulement et orientée vers la partie centrale de celui-ci, et
. une face externe qui, en position d'ouverture, est orientée vers la paroi interne de la base et dont une partie au moins, située au voisinage de chaque bord latéral, vient au contact de la paroi interne de la base en position d'ouverture et constitue ainsi un des moyens de limitation du mouvement d'ouverture,

la base ayant une forme sensiblement annulaire et comprenant elle aussi :
. un bord d'attaque, situé en amont de l'écoulement, et
. un bord de fuite, situé en aval de l'écoulement, ainsi que
. une paroi externe, destinée à recevoir des moyens de suture permettant de fixer la valve aux tissus cardiaques, et
. ladite paroi interne, dont la direction est sensiblement parallèle à l'écoulement et qui supporte les moyens de guidage en rotation et de retenue de chaque clapet,

laquelle valve cardiaque prothétique est caractérisé en ce que lesdits moyens de guidage et de retenue des clapets sont constitués, pour chaque clapet, par :
. deux arcs de guidage du bord de fuite, situés chacun au voisinage d'un des coins du clapet, chacun de ces arcs prenant naissance sur la paroi interne de la base en un point proche du bord de fuite de la base et de la zone de transition entre le bord de fuite du clapet et un des bords latéraux de ce clapet en position d'ouverture, chaque arc se projettant vers la partie centrale de la valve par une première partie de forme sensiblement circulaire, telle qu'elle reste au voisinage du bord de fuite du clapet pendant tout le mouvement de celui-ci, à l'extrémité amont de cette partie circulaire l'arc se prolongeant suivant une deuxième partie qui rejoint la paroi interne de la base en un point proche du bord d'attaque de celle-ci et servant de butée au clapet en position de fermeture, et
. au moins un arc de guidage du bord d'attaque, cet arc prenant naissance en un point de la paroi interne de la base situé près du bord d'attaque du clapet en position de fermeture, en amont de celui-ci, et ayant une forme sensiblement circulaire, telle qu'il reste au voisinage du bord d'attaque du clapet au cours de son mouvement, ledit arc de guidage du bord d'attaque se prolongeant en direction de la partie centrale de l'écoulement au moins jusqu'à l'aplomb de la face interne du clapet en position d'ouverture, l'extrémité de cet arc constituant une butée au bord d'attaque du clapet en position d'ouverture et coopérant avec la partie aval des arcs de guidage du bord de fuite et la paroi interne de la base pour éviter l'échappement du clapet en dehors de ses moyens de retenue, ladite extrémité se prolongeant jusqu'en un point au contact de la face interne du clapet en position de fermeture, constituant ainsi un point d'appui du clapet en position de fermeture.

Selon un mode de réalisation préféré de la valve cardiaque prothétique conforme à l'invention, les arcs de guidage des bords de fuite, correspondants aux coins voisins de deux clapets adjacents, se rejoignent à l'extrémité de leur partie circulaire et ont en commun la deuxième partie qui rejoint la paroi interne de la base et sert, simultanément, de butée aux deux clapets en position de fermeture.

Selon un autre mode de réalisation préféré de la valve cardiaque prothétique conforme à l'invention, les arcs de guidage du bord de fuite de chaque clapet sont constitués par les bords d'une dépression creusée dans une excroissance de la paroi interne de la base.

Selon une disposition préférée de ce mode de réalisation de la valve cardiaque prothétique, chaque excroissance de la paroi interne de la base reçoit deux dépressions correspondant aux arcs de guidage des bords de fuite de deux clapets adjacents, chacune de ces dépressions étant creusée sur une des deux faces de ladite excroissance.

Selon une autre disposition avantageuse de la valve cardiaque prothétique conforme à l'invention, les arcs de guidage du bord d'attaque de chaque clapet sont situés près d'un point du bord d'attaque de chaque clapet qui se déplace le moins au cours du mouvement du clapet.

Selon encore une autre disposition avantageuse de la valve cardiaque prothétique conforme à l'invention, le bord d'attaque de la base présente, sur toute ou partie de sa longueur, un bourrelet orienté vers le centre de l'écoulement, qui est au contact du bord d'attaque des clapets en position de fermeture et permet ainsi d'améliorer l'étanchéité de ceux-ci dans ladite position de fermeture.

Conformément à l'invention, la base de la valve cardiaque prothétique a une section sensiblement circulaire, les clapets étant au nombre de trois, ou une section sensiblement elliptique, les clapets étant alors au nombre de deux, leurs bords de fuite se rejoignant en position fermée suivant une ligne voisine du plus grand axe de ladite ellipse.

Selon encore une autre disposition avantageuse de la valve cardiaque prothétique conforme à l'invention, celle-ci comprend au moins un moyen de guidage supplémentaire pour chaque clapet, ledit moyen de guidage supplémentaire étant constitué, pour chaque clapet, par au moins une excroissance (ou déformation) de la paroi interne de la base, en un point voisin du bord d'attaque du clapet en position de fermeture et en aval de celui-ci, et disposée de telle sorte que son extrémité reste au voisinage de la face externe du clapet au cours du mouvement et sert ainsi d'appui audit clapet au cours de son pivotement.

Outre les dispositions qui précèdent, l'invention comprend encore d'autres dispositions, qui ressortiront de la description qui va suivre.

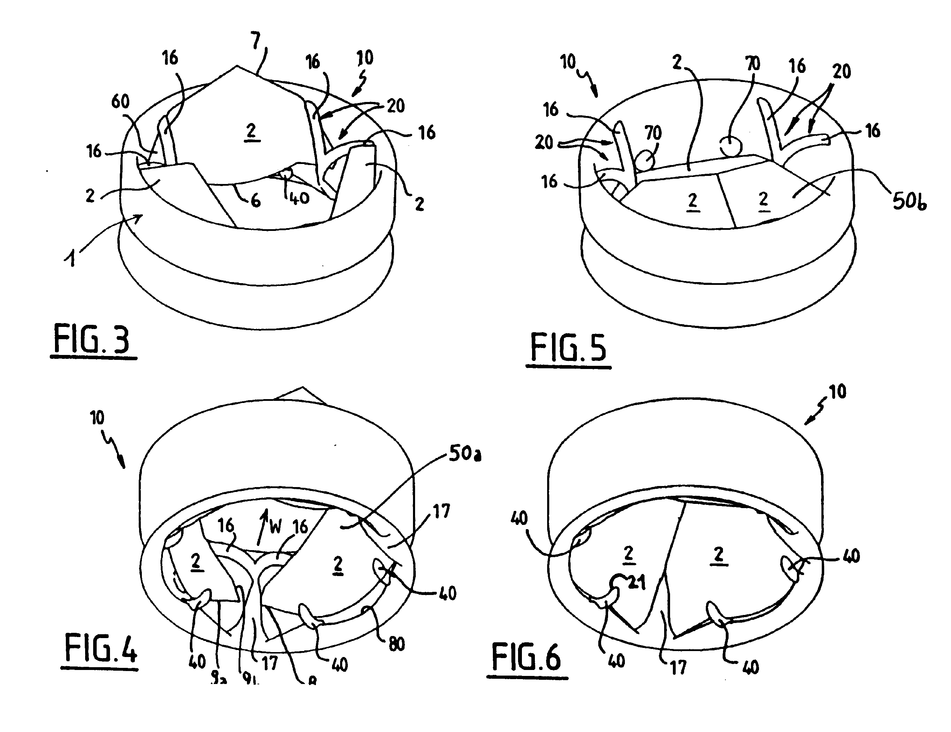

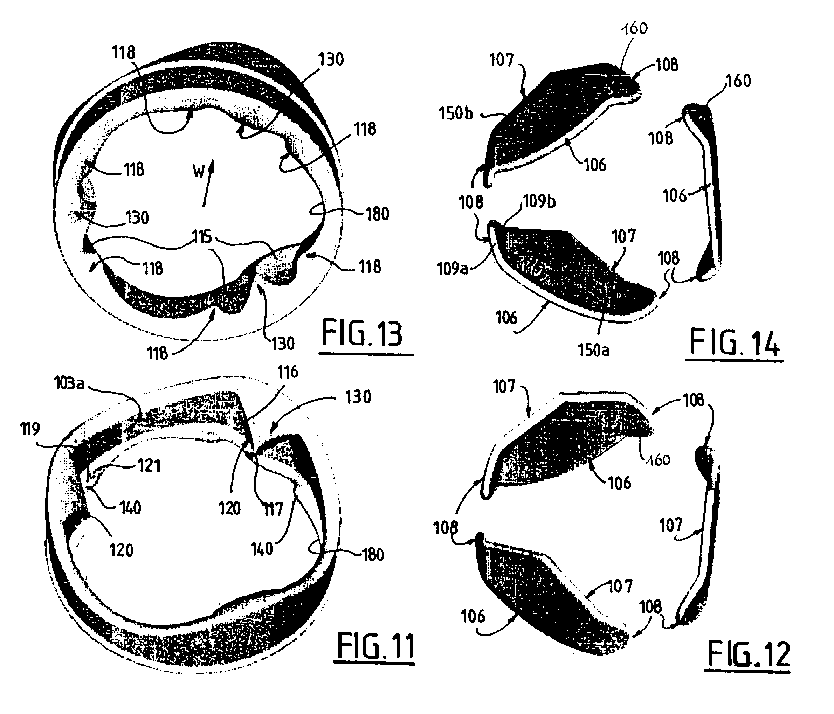

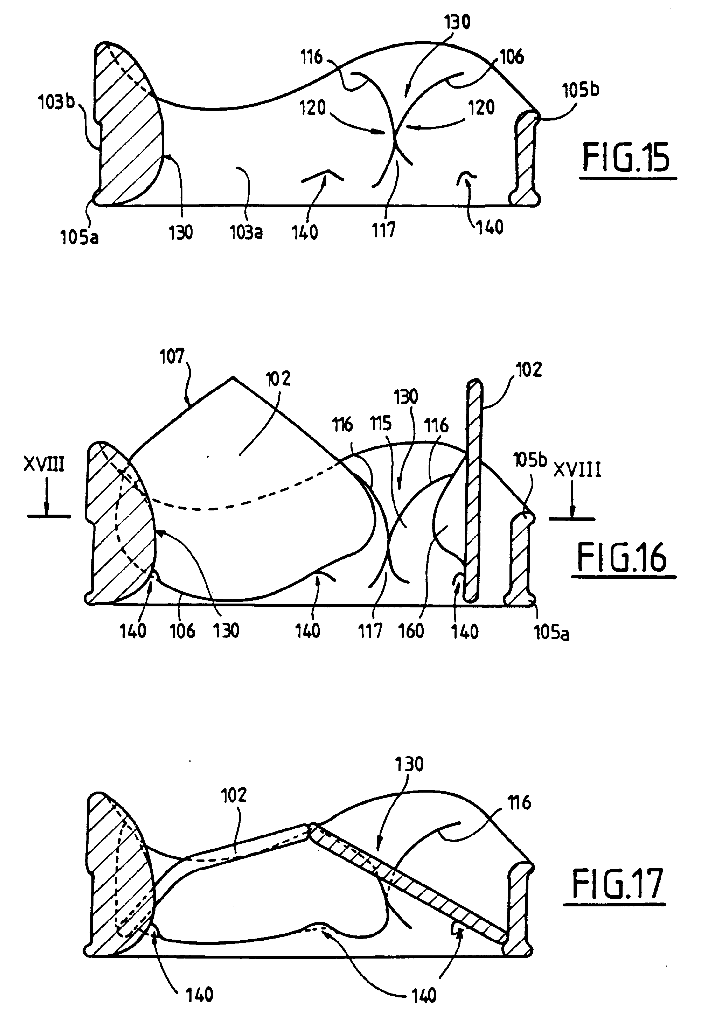

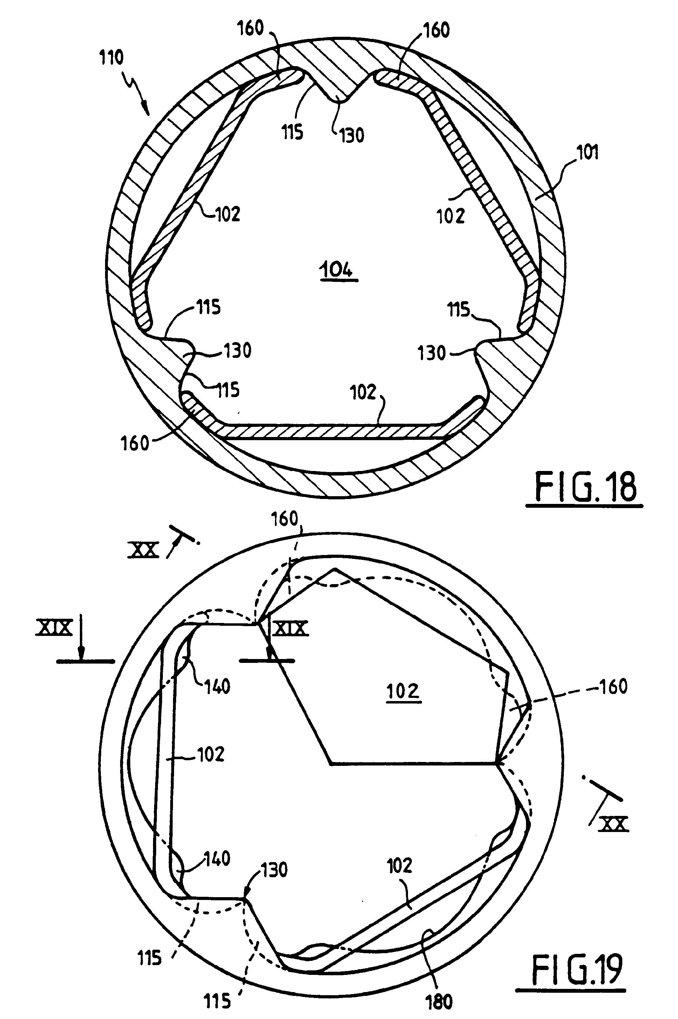

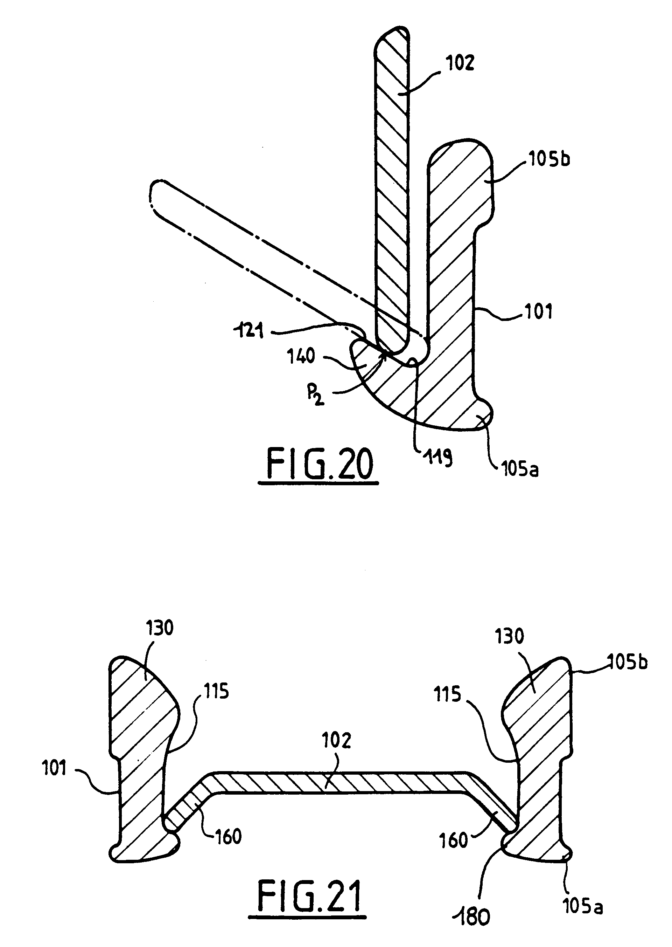

L'invention sera mieux comprise à l'aide du complément de description qui se réfère aux dessins annexés, dans lesquels :

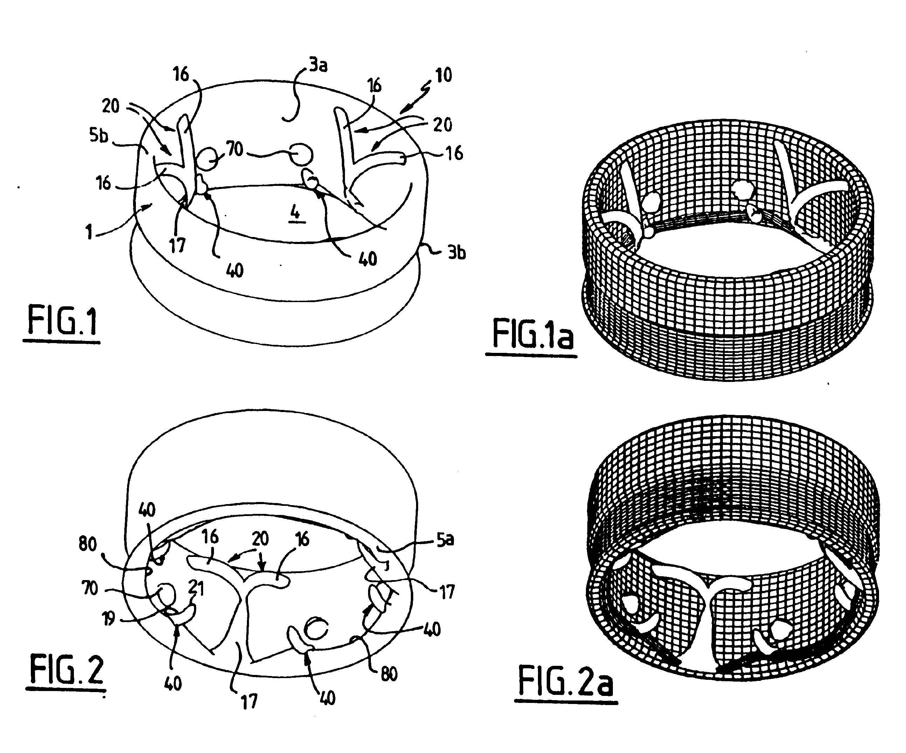

- les figures 1 à 6 sont relatives à un premier mode de réalisation de la valve cardiaque prothétique conforme à l'invention de façon plus précise :
   . les figures 1 et 2 sont des vues en perspective de dessus et de dessous, respectivement, de la seule base annulaire de support des clapets, qui ont été éliminés pour mieux apprécier la disposition des moyens de guidage et de retenue de ces mêmes clapets ;
   . les figures 1a et 2a correspondent exactement aux figures 1 et 2 sauf pour la présence de lignes visant à donner des effets d'ombre pour améliorer encore plus l'appréciation des détails se rapportant aux moyens de guidage et retenue des clapets ;
   . les figures 3 et 4 illustrent la base annulaire des figures 1 et 2 équipée de trois clapets en position d'ouverture, alors que :
   . les figures 5 et 6 illustrent la base annulaire des figures 1 et 2 avec les trois clapets en position de fermeture ;

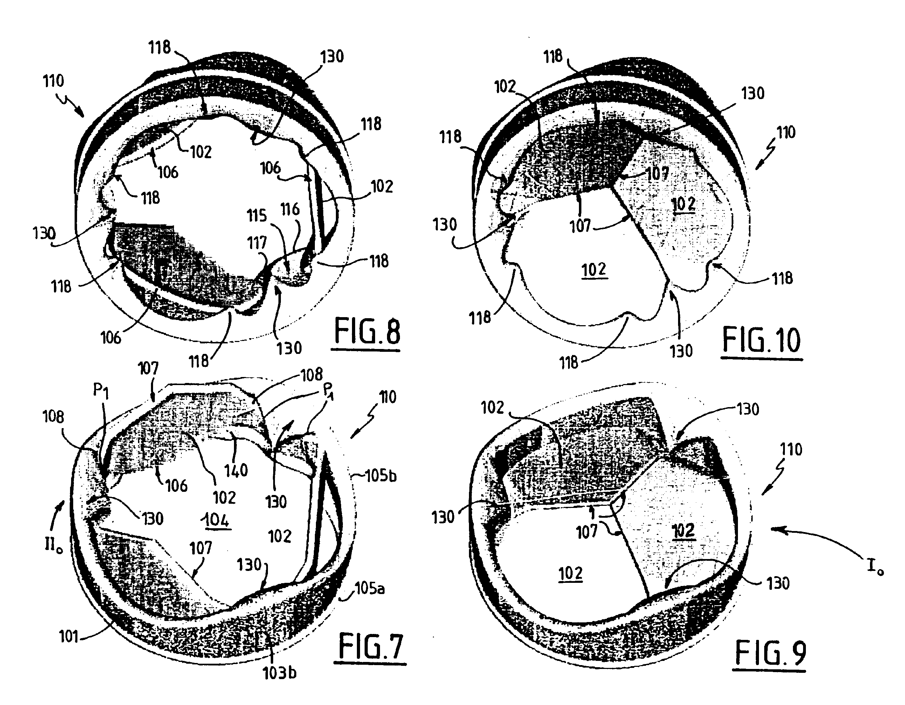

- les figures 7 à 21 sont relatives à un deuxième mode de réalisation de la valve cardiaque prothétique conforme à l'invention ; de façon plus précise :
   . la figure 7 est une vue en perspective de la valve cardiaque prothétique équipée de trois clapets en position d'ouverture, telle qu'elle est vue d'un point situé au-dessus de la valve ;
   . la figure 8 est une vue en perspective de la valve illustrée à la figure 7, lorsque le point de vue est située en dessous de la valve ;
   . la figure 9 est une vue de la même valve illustrée aux figures 7 et 8, avec les clapets en position de fermeture et avec un point de vue disposé au-dessus de la valve ;
   . la figure 10 est une vue analogue à la figure 9, qui diffère en ce que le point de vue est disposé en-dessous de la valve ;
   . les figures 11 et 12 correspondent à la figure 7 avec les éléments composant la valve prothétique illustrés de façon éclatée, à savoir avec la base annulaire de support des clapets et les clapets représentés séparément l'une par rapport aux autres ;
   . les figures 13 et 14 correspondent à la figure 8 avec les éléments composants la valve prothétique illustrés de façon éclatée, à savoir avec la base et les clapets représentés séparément l'une par rapport aux autres ;
   . la figure 15 représente une vue en coupe axiale et en élévation de la valve illustrée à la figure 11 ;
   . la figure 16 est une vue en coupe et en élévation de la valve illustrée à la figure 7 ;
   . la figure 17 est une vue en coupe axiale de la valve illustrée à la figure 9 ;
   . la figure 18 est une coupe de la valve suivant le plan XVIII de la figure 16 ;
   . la figure 19 est une vue de dessus de la valve telle qu'illustrée aux figures 7 et 9, à savoir, avec deux clapets en position d'ouverture et un clapet en position de fermeture ;
   . la figure 20 est une vue en coupe suivant le plan XIX de la figure 19 ;
   . la figure 21 est une vue en coupe suivant la plan XX de la figure 19, en correspondance du clapet représenté en position de fermeture.

Il doit être bien entendu, toutefois, que ces dessins et les parties descriptives correspondantes, sont donnés uniquement à titre d'illustration de l'objet de l'invention, dont ils ne constituent en aucune manière une limitation.

La valve cardiaque 10, illustrée aux figures 1 à 6, comporte une base annulaire 1 de support de trois clapets 2. Cette base 1 est pourvue d'une paroi externe 3b, destinée à recevoir les moyens de suture (non représentés), permettant de fixer la valve aux tissus cardiaques, et d'une paroi interne 3a, dont la direction est sensiblement parallèle à l'écoulement et qui délimite un passage central 4 destiné à l'écoulement sanguin, ainsi qu'un bord d'attaque 5a, situé en amont de l'écoulement, et un bord de fuite 5b, situé en aval de l'écoulement. Les trois clapets 2, qui sont supportés par la paroi interne 3a de la base 1, sont destinés à règler l'écoulement sanguin sous l'action de pulsations cardiaques. Le sens normal de l'écoulement sanguin est représenté à la figure 4 par une flèche W. Les clapets sont mobiles entre une position de fermeture I, telle qu'illustrée aux figures 5 et 6, et une position d'ouverture II, telle qu'illustrée aux figures 3 et 4. Lorsque les clapets sont dans la position de fermeture I, ils empêchent l'inversion de l'écoulement sanguin vers l'amont, tandis que lorsqu'ils sont dans la position d'ouverture II, ils autorisent l'écoulement sanguin vers l'aval, selon la direction illustrée par la flêche précitée W à la figure 4. Chaque clapet comporte (cf en particulier les figures 3 et 4) : un bord d'attaque 6 qui, en position de fermeture (cf en particulier les figures 5 et 6), est en contact avec la paroi interne 3 de la base annulaire 1 de support des clapets 2, alors qu'en position d'ouverture il se situe du côté amont de l'écoulement ; un bord de fuite 7 qui, en position de fermeture, est en contact (cf une fois de plus les figures 5 et 6) avec les bords de fuite des autres clapets de la valve prothétique 10, alors qu'en position d'ouverture il se situe du côté aval de l'écoulement ; deux bords latéraux (ou transversaux) 8, qui sont galbés et convexes et qui sont définis par deux profils de transition entre le bord d'attaque 6 et le bord de fuite 7. La ligne, fermée sur elle-même, définie par la succession du bord d'attaque 6, du bord de fuite 7 et des deux bords latéraux 8 de chaque clapet 2 constitue le contour de ce clapet et délimite deux faces opposées, à savoir une face interne 50a qui, en position d'ouverture, est sensiblement parallèle à l'axe de l'écoulement et orientée vers la partie centrale de celui-ci, et une face externe 50b qui, en position d'ouverture, est orientée vers la paroi interne de la base. Chaque bord latéral 8 délimite, latéralement vers l'extérieur, une zone de transition qui correspond à un coin 60 du clapet et comprend un segment amont 9a, appartenant au bord d'attaque 6, et un segment aval 9b appartenant au bord de fuite 7. Au moins une partie de la surface externe de chaque coin 60, située au voisinage du bord latéral correspondant, vient en contact avec la paroi interne de la base en position d'ouverture et constitue ainsi un des moyens de limitation du mouvement d'ouverture.

Il est possible de remarquer, aux figures 1 à 6, la présence de moyens de guidage en rotation et de retenue de chaque clapet 2 entre et dans les deux positions de fermeture et d'ouverture I et II illustrées aux figures 5-6 et 3-4, respectivement.

Ces moyens de guidage en rotation et de retenue sont constitués, pour chaque clapet, par deux arcs (ou profils) 20 de guidage et retenue du bord de fuite 7 du clapet, ainsi que par deux arcs (ou profils) 40 de guidage et retenue du bord d'attaque 6 du même clapet.

Chacun des arcs 20 de guidage du bord de fuite 7 d'un clapet 2 est situé au voisinage d'un de ses coins 60 et prend naissance sur sa paroi interne 3a en un point proche du bord de fuite 5b de la base et de la zone de transition entre le bord de fuite 7 et un des bords latéraux 8 du clapet en position d'ouverture. Chaque arc 20 se projette vers la partie centrale de la valve par une première partie 16 de forme sensiblement circulaire, telle qu'elle reste au voisinage du bord de fuite 7 du clapet pendant tout le mouvement de celui-ci.

A l'extrémité amont de cette première partie 16 chaque arc 20 se prolonge suivant une deuxième partie 17 qui rejoint la paroi interne 3a de la base en un point proche du bord d'attaque 5a de celle-ci, cette deuxième partie 17 servant de butée au clapet en position de fermeture.

Aux figures 1 à 6 chaque paire d'arcs adjacents 20 de guidage des bords de fuite 7 de deux clapets adjacents 2 comporte les premières parties précitées 16 qui se rejoignent à leur extrémité amont et qui se prolongent vers l'amont dans une deuxième partie 17 qui est commune aux deux arcs adjacents 20 et qui sert donc simultanément de butée aux deux clapets adjacents 2 en position de fermeture.

En ce qui concerne les deux arcs 40 de guidage et retenue du bord d'attaque 6 d'un clapet 2, ceux-ci prennent naissance en un point de la paroi interne 3a de la base 1 situé près du bord d'attaque 6 du clapet en position de fermeture, en amont de celui-ci. Chaque arc 40 comporte une première portion 19 ayant une forme sensiblement circulaire, telle qu'il reste au voisinage du bord d'attaque 6 du clapet au cours de son mouvement, qui se prolonge en direction de la partie centrale de l'écoulement au moins jusqu'à l'aplomb de la face interne 50a du clapet en position d'ouverture. L'extrémité radialement interne de chaque portion 9 constitue ainsi une butée pour le bord d'attaque du clapet correspondant en position d'ouverture et coopère avec la partie aval 16 des arcs de guidage du bord de fuite 7 et la paroi interne 3a de la base pour éviter que le clapet ne s'échappe en dehors de ses moyens de retenue.

L'extrémité radialement interne de la portion 19 de chaque arc 40 se prolonge suivant une deuxième portion 21 jusqu'en un point qui est au contact de la face interne du clapet en position de fermeture, constituant ainsi un point d'appui du clapet dans cette position.

Aux figures 1 à 6 (cf en particulier les figures 1 et 2) on a également représenté des moyens de guidage supplémentaires pour chaque clapet qui peuvent être présents sur la paroi interne 3a de la base 1. Il s'agit de 2 excroissances (ou déformations) 70 de cette paroi situées en un point voisin du bord d'attaque 6 du clapet en position de fermeture et en aval de ce point. Ces excroissances 70 sont disposées de telle sorte que leur extrémité reste au voisinage de la face externe 50b du clapet, tout en servant ainsi d'appui au clapet au cours du mouvement de celui-ci.

Le bord d'attaque 5a de la base 1 présente sur sa longueur une lèvre (ou un bourrelet) 80 orientée vers le centre de l'écoulement, qui est en contact avec le bord d'attaque 6 des clapets 2 en position de fermeture et qui permet ainsi d'améliorer l'étanchéité de ceux-ci dans leur position de fermeture.

La valve cardiaque 110, illustrée aux figures 7 à 21, comporte une base annulaire 101 de support de trois clapets 102. Cette base 101 est pourvue d'une paroi externe 103b, destinée à recevoir les moyens de suture (non représentés), permettant de fixer la valve aux tissus cardiaques, et d'une paroi interne 103a, dont la direction est sensiblement parallèle à l'écoulement et qui délimite un passage central 104 destiné à l'écoulement sanguin, ainsi qu'un bord d'attaque 105a, situé en amont de l'écoulement, et un bord de fuite 105b situé en aval de l'écoulement. Les trois clapets 102, qui sont supportés par la paroi interne 103a de la base 101, sont destinés à régler l'écoulement sanguin sous l'action de pulsations cardiaques. Le sens normal de l'écoulement sanguin est représenté à la figure 13 par une flèche W. Les clapets sont mobiles entre une position de fermeture Iₒ, telle qu'illustrée aux figures 9 et 10, et une position d'ouverture IIₒ, telle qu'illustrée aux figures 7 et 8. Lorsque les clapets sont dans la position de fermeture Iₒ, ils empêchent l'inversion de l'écoulement sanguin vers l'amont, tandis que lorsqu'ils sont dans la position d'ouverture IIₒ, ils autorisent l'écoulement sanguin vers l'aval, selon la direction illustrée par la flèche précitée W à la figure 13. Chaque clapet comporte (cf en particulier les figures 12 et 14) : un bord d'attaque 106 qui, en position de fermeture (cf en particulier les figures 9 et 10) est en contact avec la paroi interne 103a de la base annulaire 101 de support des clapets 102, alors qu'en position d'ouverture il se situe du côté amont de l'écoulement un bord de fuite 107 qui, en position de fermeture, est au contact (cf une fois de plus les figures 9 et 10) des bords de fuite des autres clapets de la valve prothétique 101, alors qu'en position d'ouverture il se situe du côté aval de l'écoulement ; deux bords latéraux (ou transversaux) 108, qui sont galbés et convexes et qui sont définis par deux profils de transition entre le bord d'attaque 106 et le bord de fuite 107. La ligne, fermée sur elle-même, définie par la succession du bord d'attaque 106, du bord de fuite 107 et des deux bords latéraux 108 de chaque clapet 102 constitue le contour de ce clapet et délimite deux faces opposées, à savoir une face interne 150a qui, en position d'ouverture, est sensiblement parallèle à l'axe de l'écoulement et orientée vers la partie centrale de celui-ci, et une face externe 150b qui, en position d'ouverture, est orienté vers la paroi interne de la base. Chaque bord latéral 108 délimite, latéralement vers l'extérieur, une zone de transition qui correspond à un coin 160 du clapet et comprend un segment amont 109a, appartenant au bord d'attaque 106, et un segment aval 109b appartenant au bord de fuite 107. Au moins une partie de la surface externe de chaque coin 160, située au voisinage du bord latéral correspondant, vient en contact avec la paroi interne 103a de la base en position d'ouverture et constitue ainsi un des moyens de limitation du mouvement d'ouverture.

Il est possible de remarquer, aux figures 7 à 21, la présence de moyens de guidage en rotation et de retenue de chaque clapet 102 entre et dans les deux positions de fermeture et d'ouverture Iₒ et IIₒ illustrées aux figures 9-10 et 7-8, respectivement.

Ces moyens de guidage en rotation et de retenue sont constitués, pour chaque clapet, par deux arcs (ou profils) 120 de guidage et retenue du bord de fuite 107 du clapet, ainsi que par deux arcs (ou profils) 140 de guidage et retenue du bord d'attaque 106 du même clapet.

Chaque arc 120 de guidage et de retenue du bord de fuite 107 d'un clapet 102 est ménagé dans une excroissance 130, radialement interne, de la paroi interne 103a de la base 101 de support des clapets 102. Le nombre d'excroissances 130, dont est pourvue la base 101 de la valve prothétique 110, est égal à trois dans les figures, de manière à permettre le guidage en rotation et la retenue des trois clapets, chaque clapet 102 étant compris entre une paire d'excroissances 130. Chaque excroissance 130 comporte une première et une deuxième dépressions identiques 115, qui sont orientées de telle manière que les dépressions 115 correspondant aux coins 160 d'un même clapet 102 se font face. Les bords de chacune de ces dépressions constituent des moyens de guidage et retenue au clapet, à savoir :
- une portion radialement externe, constituée par le prolongement de la paroi interne 103a de la base 101, laquelle portion vient au contact de la surface externe du coin 160 du clapet correspondant en position d'ouverture, et
- une portion radialement interne, correspondant à un des arcs 120 de guidage et retenue du bord de fuite 107 d'un clapet 102 et comprenant:
   . un segment aval 116, galbé et convexe, de guidage du mouvement d'un point P1 (cf notamment la figure 7) appartenant au bord de fuite 107 du clapet 102 et situé sur le segment aval 109b du bord latéral 108 correspondant du clapet, et ce lorsque le clapet passe de la position d'ouverture IIₒ à la position de fermeture Iₒ et vice versa, et
   . un segment amont 117, lui aussi galbé et convexe, de retenue, à savoir d'appui du même point P1 précité, et ce lorsque le clapet 102 est en position de fermeture. Bien entendu, chaque dépression 115 présente un profil qui s'adapte au mouvement d'un des bords latéraux 108 de chaque clapet 102 lorsque celui-ci passe de la position d'ouverture IIₒ à la position de fermeture Iₒ et vice versa.

Il est donc clair que dans le mode de réalisation illustré aux figures 7 à 21 les arcs 120 de guidage du bord de fuite de chaque clapet sont constitués par les bords délimitant chacune des dépressions correspondantes ménagées dans les excroissances de la paroi interne de la base.

Chaque arc 140 de guidage et de retenue du bord 106 de chaque clapet 102 est constitué par la partie aval d'une excroissance 118, radialement interne, de la paroi interne 103a de la base de support 101.

Cette excroissance 118 comprend :
. un segment interne 119 de guidage du mouvement d'un point P2 (cf la figure 20) du bord d'attaque 106 du clapet 102, lorsque celui-ci passe de la position d'ouverture IIₒ à la position de fermeture Iₒ, et
. un segment externe 121 de retenue, à savoir d'appui de ce même point P2, lorsque le clapet 102 passe de la position de fermeture Iₒ à la position d'ouverture IIₒ. Ce segment externe 121 du profil galbé, de guidage et retenue du bord d'attaque 106 du clapet 102 se prolonge légèrement au-delà de la l'aplomb du clapet 102, lorsque celui-ci est en position d'ouverture IIₒ.

Une lèvre (ou un bourrelet) d'appui 180 du bord d'attaque 106 du clapet 102 peut être également ménagée dans ce cas sur le bord d'attaque 105a de la base 101 de la valve prothétique 110, comme illustré clairement en particulier à la figure 11, où cette lèvre 180 relie deux excroissances 118 consécutives entre elles.

Il y a lieu de souligner que dans chacun des modes de réalisation illustrés aux figures 1 à 6 et 7 à 21, respectivement, les arcs de guidage 40 et 140, respectivemnt du bord d'attaque de chaque clapet sont situés près d'un point du bord d'attaque de chaque clapet qui décrit le déplacement le plus court au cours du mouvement du clapet.

La valve 110, conforme à la présente invention, peut être également pourvue de moyens d'avance à la fermeture (à savoir de moyens destinés à anticiper la fermeture des clapets sur le retour de l'écoulement sanguin en sens inverse et à minimiser ainsi les problèmes de régurgitation), tels que décrits dans les Brevets français et américains aux noms des Demandeurs déjà évoqués plus haut. Il s'agit essentiellement de masses magnétiques (non représentées, parce que décrites dans les Brevets précités), qui sont incorporées dans chaque clapet de manière que les coins de deux clapets contigus présentent des polarités magnétiques de signes opposés, pouvant coopérer avec des masses magnétiques supplémentaires disposées dans la base de la valve prothétique entre deux clapets contigus, et dont l'encombrement est égal à la distance qui existe entre les coins adjacents de clapets contigus en position d'ouverture. Chacune de ces masses magnétiques supplémentaires est disposée dans la base de manière que ces polarités correspondent aux polarités de signes opposés des masses magnétiques ménagées dans les coins des clapets.

Bien sûr, les masses magnétiques précitées sont calculées pour que l'attraction entre les coins de clapets voisins, -visant à fermer la valve-, s'oppose exactement aux forces de pression et de frottement qui tendent à ouvrir les clapets lorsque la vitesse de l'écoulement en sens direct atteint un certain pourcentage de sa valeur maximale. Cela signifie que, grâce aux moyens d'avance à la fermeture, celle-ci s'amorcera avant le retour de l'écoulement.

En ce qui concerne la réalisation de ces masse magnétiques, celles-ci peuvent être constituées, de façon connue des techniciens en la matière, par des petits aimants fixés sur les coins des clapets et sur la base par tous moyens appropriés, ou par un matériau magnétique incorporé lors de la fabrication de la valve prothétique, sous forme finement dispersée dans les coins des clapets et dans la base en des positions appropriées, telles qu'illustrées aux figures 2, 3 et 3a des Brevets français et américain évoqués plus haut, aux noms des Demandeurs.

En ce qui concerne les figures 15 à 21 (qui sont des vues en coupe pour la plupart, sauf la figure 19 qui est une vue de dessus), celles-ci permettent d'apprécier mieux les détails de réalisation de la valve prothétique selon l'invention, telle qu'illustrée en perspective aux figures 7 à 14 et que décrite précédemment.

Il va de soi que la valve cardiaque prothétique 10 conforme à la présente invention répond aussi à toutes les dispositions qui ont été évoquées dans les deux Brevets français et américains, au nom des Demandeurs, rappelés à plusieurs reprises dans le corps de cette description, y compris aussi les indications concernant le choix le plus approprié des matériaux susceptibles d'être utilisés pour la construction de la valve, notamment en ce qui concerne le choix préférentiel du carbone pyrolytique. Il est facile de vérifier que la configuration des moyens de guidage et de retenue des clapets de la valve prothétique, conforme à l'invention, se prête particulièrement à une fabrication permettant le revêtement avec du carbone pyrolytique, non seulement de la base de la prothèse valvulaire mais également des excroissances 118 et 130, dans lesquelles sont ménagés les profils de guidage en rotation et de retenue des bords d'attaque et de fuite de chaque clapet.

Il y a en outre lieu de remarquer que chacune des dépressions 115, ménagées dans les excroissances 130, diffère, à proprement parler, d'une des cavités de la valve prothétique objet des Brevets FR-2 407 709 et US-SN 847 780, évoqués plus haut, en ce sens qu'il n'existe aucun point du coin correspondant du clapet qui reste à un endroit fixe de la dépression 115 au cours du mouvement. Cette condition de fonctionnement, qui permet un balayage complet de la dépression à chaque cycle et évite ainsi tout risque de stagnation, n'est permise que grâce à la coopération avec les arcs de guidage et retenue 140 du bord d'attaque 106 de chaque clapet 102.

## Revendications

1. Valve cardiaque prothétique (10 ; 110) comprenant une base (1 ; 101) et au moins deux clapets (2 ; 102), chaque clapet comprenant :
. un bord d'attaque (6 ; 106) qui , en position de fermeture, est au contact de la paroi interne (3a ; 103a) de la base (1 ; 101) et en position d'ouverture se situe du côté amont de l'écoulement,
. un bord de fuite (7 ; 107) qui, en position de fermeture, est au contact du ou des bords de fuite du ou des autres clapets (2 ; 102) et, en position d'ouverture, se situe du côté aval de l'écoulement,
. deux bords latéraux (8 ; 108) constituant des profils de transition entre le bord d'attaque (6 ; 106) et le bord de fuite (7 ; 107) de chaque clapet (2 ; 102), chaque bord latéral (8 ; 108) délimitant, latéralement vers l'extérieur, une zone de transition qui correspond à un coin (60 ; 160) du clapet et comprenant un segment amont (9a ; 109a), appartenant au bord d'attaque (6 ; 106), et un segment aval (9b ; 109b) appartenant au bord de fuite,
la ligne définie par la succession du bord d'attaque (6 ; 106), du bord de fuite (7 ; 107) et des bords latéraux (8 ; 108) de chaque clapet (2 ; 102) constituant le contour de ce clapet et délimitant deux faces opposées, dont :
. une face interne (50a ; 150a) qui, en position d'ouverture, est sensiblement parallèle à l'axe de l'écoulement et orientée vers la partie centrale de celui-ci, et
. une face externe (50b ; 150b) qui, en position d'ouverture, est orientée vers la paroi interne de la base (1 ; 101) et dont une partie au moins, située au voisinage de chaque bord latéral (8 ; 108), vient au contact de la paroi interne (3a ; 103a) de la base (1 ; 101) en position d'ouverture et constitue ainsi un des moyens de limitation du mouvement d'ouverture,
la base (1 ; 101) ayant une forme sensiblement annulaire et comprenant elle aussi :
. un bord d'attaque (5a ; 105a), situé en amont de l'écoulement, et
. un bord de fuite (5b ; 105b), situé en aval de l'écoulement, ainsi que
. une paroi externe (3b ; 103b), destinée à recevoir des moyens de suture permettant de fixer la valve (10 ; 110) aux tissus cardiaques, et
. ladite paroi interne (3a ; 103a), dont la direction est sensiblement parallèle à l'écoulement et qui supporte les moyens de guidage en rotation et de retenue de chaque clapet (2 ; 102),
laquelle valve cardiaque prothétique (10 ; 110) est caractérisé en ce que lesdits moyens de guidage et de retenue des clapets sont constitués, pour chaque clapet (2 ; 102), par :
. deux arcs de guidage (20 ; 120) du bord de fuite (7 ; 107), situés chacun au voisinage d'un des coins (60 ; 160) du clapet (2 ; 102), chacun de ces arcs (20 ; 120) prenant naissance sur la paroi interne (3a ; 103a) de la base (1 ; 101) en un point proche du bord de fuite (5b ; 105b) de la base et de la zone de transition entre le bord de fuite du clapet et un des bords latéraux (8 ; 108) de ce clapet (2 ; 102) en position d'ouverture, chaque arc (20 ; 120) se projettant vers la partie centrale de la valve (10 ; 110) par une première partie (16 ; 116) de forme sensiblement circulaire, telle qu'elle reste au voisinage du bord de fuite (7 ; 107) du clapet (2 ; 102) pendant tout le mouvement de celui-ci, à l'extrémité amont de cette partie circulaire (16 ; 116) l'arc (20 ; 120) se prolongeant suivant une deuxième partie (17 ; 117) qui rejoint la paroi interne (3a ; 103a) de la base (1 ; 101) en un point proche du bord d'attaque (5a ; 105a) de celle-ci et servant de butée au clapet (2 ; 102) en position de fermeture, et
. au moins un arc de guidage (40 ; 140) du bord d'attaque (6 ; 106), cet arc (40 ; 140) prenant naissance en un point de la paroi interne (3a ; 103a) de la base (1 ; 101) situé près du bord d'attaque (6 ; 106) du clapet (2 ; 102) en position de fermeture, en amont de celui-ci, et ayant une forme sensiblement circulaire, telle qu'il reste au voisinage du bord d'attaque (6 ; 106) du clapet (2 ; 102) au cours de son mouvement, ledit arc de guidage (40 ; 140) du bord d'attaque (6 ; 106) se prolongeant en direction de la partie centrale de l'écoulement au moins jusqu'à l'aplomb de la face interne (50a ; 150a) du clapet (2 ; 102) en position d'ouverture, l'extrémité de cet arc constituant une butée au bord d'attaque (6 ; 106) du clapet (2 ; 102) en position d'ouverture et coopérant avec la partie aval (16 ; 116) des arcs de guidage (20 ; 120) du bord de fuite (7 ; 107) et la paroi interne (3a ; 103a) de la base (1 ; 101) pour éviter l'échappement du clapet (2 ; 102) en dehors de ses moyens de retenue, ladite extrémité se prolongeant jusqu'en un point au contact de la face interne (50a ; 150a) du clapet (2 ; 102) en position de fermeture, constituant ainsi un point d'appui du clapet (2 ; 102) en position de fermeture.

2. Valve cardiaque prothétique (10) selon la revendication 1, caractérisée en ce que les arcs de guidage (20) des bords de fuite (7), correspondants aux coins (60) voisins de deux clapets (2) adjacents, se rejoignent à l'extrémité de leur partie circulaire (16) et ont en commun la deuxième partie (17) qui rejoint la paroi interne (3a) de la base (1) et sert, simultanément, de butée aux deux clapets (2) en position de fermeture.

3. Valve cardiaque prothétique (110) selon la revendication 1, caractérisée en ce que les arcs de guidage (120) du bord de fuite (107) de chaque clapet (102) sont constitués par les bords d'une dépression (115) creusée dans une excroissance (130) de la paroi interne (103a) de la base (101).

4. Valve cardiaque prothétique (110) selon la revendication 3, caractérisée en ce que chaque excroissance (130) de la paroi interne (103a) de la base (101) reçoit deux dépressions (115) correspondant aux arcs de guidage (120) des bords de fuite (107) de deux clapets (102) adjacents, chacune de ces dépressions (115) étant creusée sur une des deux faces de ladite excroissance (130).

5. Valve cardiaque prothétique (10 ; 110) selon l'une quelconque des revendications 1 à 4, caractérisée en ce que les arcs de guidage (40 : 140) du bord d'attaque (6 ; 106) de chaque clapet (2 ; 102) sont situés chacun près d'un point du bord d'attaque de chaque clapet qui se déplace le moins au cours du mouvement du clapet.

6. Valve cardiaque prothétique (10 ; 110) selon l'une quelconque des revendications 1 à 5, caractérisée en ce que le bord d'attaque (5a ; 105a) de la base (1 ; 101) présente, sur toute ou partie de sa longueur, un bourrelet orienté vers le centre de l'écoulement, qui est au contact du bord d'attaque des clapets (2 ; 102) en position de fermeture et permet ainsi d'améliorer l'étanchéité de ceux-ci dans ladite position de fermeture.

7. Valve cardiaque prothétique (10 ; 110) selon l'une quelconque des revendications 1 à 6, caractérisée en ce que la base (1 ; 101) a une section sensiblement circulaire, et en ce que les clapets (2 ; 102) sont au nombre de trois.

8. Valve cardiaque prothétique selon l'une quelconque des revendications 1 à 6, caractérisée en ce que la base a une section sensiblement elliptique, et en ce que les clapets sont au nombre de deux, leurs bords de fuite se rejoignant en position fermée suivant une ligne voisine du plus grand axe de ladite ellipse.

9. Valve cardiaque prothétique (10) selon l'une quelconque des revendications 1 à 8, caractérisée en ce que celle-ci comprend au moins un moyen de guidage supplémentaire pour chaque clapet (2), ledit moyen de guidage supplémentaire étant constitué, pour chaque clapet (2), par au moins une excroissance (ou déformation, 70) de la paroi interne (3a) de la base (1), en un point voisin du bord d'attaque (6) du clapet (2) en position de fermeture et en aval de celui-ci, et disposée de telle sorte que son extrémité reste au voisinage de la face externe (50b) du clapet (2) au cours du mouvement et sert ainsi d'appui audit clapet (2) au cours de son pivotement.

10. Valve cardiaque prothétique selon l'une quelconque des revendications 1 à 9, caractérisée en ce que chacun des clapets (2 ; 102) est pourvu de moyens d'avance à la fermeture, à savoir de moyens destinés à anticiper la fermeture des clapets sur le retour de l'écoulement sanguin en sens inverse.

11. Valve cardiaque prothétique selon la revendication 10, caractérisée en ce que les dits moyens d'avance à la fermeture sont constitués par des masses magnétiques de polarités opposées ménagées au niveau des coins de chaque clapet (2 ; 102), les polarités magnétiques des coins de deux clapets contigus étant opposées.

12. Valve cardiaque prothétique selon la revendication 11, caractérisée en ce que lesdites masses magnétiques ménagées au niveau des coins (60 ; 160) de chaque clapet (2 ; 102) coopèrent avec des masses magnétiques supplémentaires qui sont disposées dans la base (1 ; 101) entre deux clapets contigus et dont l'encombrement est sensiblement égal à la distance qui existe entre les coins adjacents de deux clapets contigus en position d'ouverture, chaque masse magnétique supplémentaire étant disposée dans la base de manière que ses polarités correspondent aux polarités de signe opposé des masses magnétiques ménagées dans les coins des clapets.

13. Valve cardiaque prothétique selon l'une quelconque des revendications 11 et 12, caractérisée en ce que lesdites masses magnétiques sont constituées par des petits aimants.

14. Valve cardiaque prothétique selon l'une quelconque des revendications 11 et 12, caractérisée en ce que lesdites masses magnétiques sont constituées par un matériau magnétique incorporé lors de la fabrication de chaque clapet (2 ; 102) ou de la base (1 ; 101).

## Claims

1. Prosthetic heart valve (10; 110), comprising a base (1; 101) and at least two flap valves (2; 102), each flap valve comprising :
. a leading edge (6; 106) which, in the closed position, is in contact with the internal wall (3a; 103a) of the base (1; 101) and in the open position is situated on the upstream side of the flow,
. a trailing edge (7; 107) which, in the closed position, is in contact with the trailing edge(s) (7; 107) of the other valve(s) (2; 102) and, in the open position,is situated on the downstream side of the flow,
. two lateral edges (8; 108) forming transition profiles between the leading edge (6; 106) and the trailing edge (7; 107) of each flap valve (2; 102), each lateral edge (8; 108) defining, laterally outwardly, a transition zone which corresponds to a corner (60; 160) of the flap valve and comprising an upstream segment (9a; 109a), belonging to the leading edge (6; 106) and a downstream segment (9b; 109b) belonging to the trailing edge,
the line defined by the succession of the leading edge (6; 106), the trailing edge (7; 107) and the lateral edges (8; 108) of each flap valve (2; 102) forming the contour of this flap valve and defining two opposite faces, including :
. an internal face (50a; 150a) which, in the open position, is substantially parallel to the axis of the flow and oriented to the central portion thereof, and
. an external face (50b; 150b) which, in the open position, is oriented towards the internal wall of the base (1; 101) and a part of which at least, situated in the vicinity of each lateral edge (8; 108), comes into contact with the internal wall (3a; 103a) of the base (1; 101) in the open position and thus forms a means for limiting the opening movement,
the base (1; 101) having a substantially annular form and comprising also :
. a leading edge (5a; 105a) situated upstream of the flow, and
. a trailing edge (5b; 105b) situated downstream of the flow and
. an external wall (3b; 103b) for receiving the suture means for fixing the valve (10; 110) to the heart tissues, and
. said internal wall (3a; 103a), whose direction is substantially parallel to the flow and which supports the means guiding in rotation and retaining each flap valve (2; 102),
which prosthetic heart valve (10; 110) is characterized in that said means for guiding and retaining the flap valves are formed, for each flap valve (2; 102) by :
. two arcs (20; 120) guiding the trailing edge (7; 107), each situated in the vicinity of one of the corners (60; 160) of the flap valves (2; 102), each of these arcs (20; 120) originating on the internal wall (3a; 103a) of the base (1; 101) at a point close to the trailing edge (5b; 105b) of the base and of the transition zone between the trailing edge of the flap valve and one of the lateral edges (8; 108) of this flap valve (2; 102) in the open position, each arc (20; 120) projecting towards the central portion of the valve (10; 110) by a first portion (16; 116) of a substantially circular form, such that it rests in the vicinity of the trailing edge (7; 107) of the flap valve (2; 102) during the whole movement thereof, at the upstream end of the circular portion (16; 116) the arc (20; 120) extending along a second portion (17; 117) which joins the internal wall (3a; 103a) of the base (1; 101) at a point close to the leading edge (5a; 105a) thereof and serving as stop for the flap valve (2; 102) in the closed position, and
. at least one arc (40; 140) guiding the leading edge (6; 106), this arc (40; 140) originating at a point of the internal wall (3a; 103a) of the base situated close to the leading edge (6; 106) of the flap valve (2; 102) in the closed position, upstream thereof, and having a substantially circular shape, such that it remains in the vicinity of the leading edge (6; 106) of the flap valve (2; 102) during its movement, said arc (40; 140) guiding the leading edge (6; 106) extending in the direction of the central portion of the flow at least as far as to be in line with the internal face (50a; 150a) of the flap valve (2; 102) in the open position, the end of this arc forming a stop for the leading edge (6; 106) of the flap valve (2; 102) in the open position and cooperating with the downstream portion (16; 116) of the guide arcs (20; 120) of the trailing edge (7; 107) and the internal wall (3a; 103a) of the base (1; 101) to prevent the flap valve (2; 102) escaping out of the retaining means, said end extending as far as a point in contact with the internal face (50a; 150a) of the flap valve (2; 102) in the closed position, thus forming a bearing point for the flap valve (2; 102) in the closed position.

2. Prosthetic heart valve (10) according to claim, characterized in that the arcs (20) guiding the trailing edges (7) corresponding to the adjacent corners (60) of two adjacent flap valves (2) join up at the end of their circular portion (16) and have in common the second portion (17) which joins the internal wall (3a) of the base (1) and serves simultaneously as stop for both flap valves (2) in the closed position.

3. Prosthetic heart valve (110) according to claim 1, characterized in that the arcs (120) guiding the trailing edge (107) of each flap valve (102) are formed by the edges of a depression (15) formed in an excrescence (130) of the internal wall (103a) of the base (101).

4. Prosthetic heart valve (110) according to claim 3, characterized in that each excrescence (130) of the internal wall (103a) of the base (101) receives two depressions (115) corresponding to the guide arcs (120) of the trailing edges (107) of two adjacent flap valve (102), each of these depressions (115) being formed in one of the two faces of said excrescence (130).

5. Prosthetic heart valve (10; 110) according to any one of claims 1 to 4, characterized in that the guide arcs (40; 140) of the leading edge (6; 106) of each flap valve (2; 102) are each situated close to a point of the leading edge of each flap valve which moves the least during the movement of the flap valve.

6. Prosthetic heart valve (10; 110) according to any one of claims 1 to 5, characterized in that the leading edge (5a; 105a) of the base (101) has, over all or part of its length, a bead oriented towards the centre of the flow, which is in contact with the leading edge of the flap valves (2; 102) in the closed position and thus improves the sealing thereof in said closed position.

7. Prosthetic heart valve (10; 110) according to any one of claims 1 to 6, characterized in that the base (101) has a substantially circular cross section and in that the flap valves (2; 102) are three in number.

8. Prosthetic heart valve according to any one of claims 1 to 6, characterized in that the base has a substantially elliptic cross section and in that the flap valves are two in number, their trailing edges joining up in the closed position along a line close to the largest axis of said ellipsis.

9. Prosthetic heart valve (10) according to any one of claims 1 to 8, characterized in that it comprises at least one additional guide means for each flap valve (2), said additional guide means being formed, for each flap valve (2), by at least one excrescence (or deformation 70) of the internal wall (3a) of the base (1), at a point close to the leading edge (6) of the flap valve (2) in the closed position and downstream thereof and disposed so that its end remains in the vicinity of the external face (50b) of the flap valve (2) during the movement and thus serves as bearing point for said flap valve (2) during its pivoting movement.

10. Prosthetic heart valve according to any one of claims 1 to 9, characterized in that each of the flap valves (2; 102) is provided with closure advance means, namely means anticipating closure of the flap valves in the blood flow return in the opposite direction.

11. Prosthetic heart valve according to claim 10, characterized in that said closure advance means are formed by magnetic masses of opposite polarities formed at the level of the corners of each flap valve (2; 102), the magnetic polarities of the corners of two contiguous flap valves being opposite.

12. Prosthetic heart valve according to claim 11, characterized in that said magnetic masses formed at the level of the corners (60; 160) of each flap valve (2; 102) cooperate with additional magnetic masses which are disposed in the base (1; 101) between two contiguous flap valves and whose size is substantially equal to the distance which exists between the adjacent corners of two contiguous flap valves in the open position, each additional magnetic mass being disposed in the base so that its polarities correspond to the opposite polarities of the magnetic masses formed in the corners of the flap valves.

13. Prosthetic heart valve according to any one of claims 11 and 12, characterized in that said magnetic masses are formed by small magnets.

14. Prosthetic heart valve according to any one of claims 11 and 12, characterized in that said magnetic masses are formed by a magnetic material incorporated during manufacture of each flap valve (2; 102) or of the base (1; 101).

## Patentansprüche

1. Herzklappenprothese (10; 110) mit einer Basis (1; 101) und zumindest zwei Verschlußklappen (2; 102), wobei jede Verschlußklappe aufweist:
eine Vorderkante (6; 106), die sich in Schließstellung mit der Innenwand (3a; 103a) der Basis (1; 101) in Berührung befindet und in Offenstellung stromaufseitig der Strömung liegt,
eine Hinterkante (7; 107), die sich in Schließstellung mit der oder den Hinterkanten der oder der anderen Verschlußklappen (2; 102) in Berührung befindet und in Offenstellung stromabseitig der Strömung liegt, zwei Seitenkante (8; 108), die zwischen der Vorderkante (6; 106) und der Hinterkante (7; 107) jeder Verschlußklappe (2; 102) Übergangsprofile bilden, wobei jede Seitenkante (8; 108) seitlich nach außen einen Übergangsbereich abgrenzt, der einer Ecke (60; 160) der Verschlußklappe entspricht und einen stromauf liegenden Abschnitt (9a; 109a), welcher der Vorderkante (6; 106) gehört und einen stromab liegenden Abschnitt (9b, 109b), welcher der Hinterkante gehört, aufweist, wobei die Linie, die durch die Aufeinanderfolge der Vorderkante (6; 106), der Hinterkante (7;107) und der Seitenkanten (8; 108) jeder Verschlußklappe (2; 102) definiert ist, die Kontur dieser Verschlußklappe bildet und zwei gegenüberliegende Flächen abgrenzt, unter ihnen:
eine Innenfläche (50a; 150a), die in Offenstellung im wesentlichen parallel zu der Achse der Strömung liegt und gegen ihren zentralen Teil dieser gerichtet ist und
eine Außenfläche (50b; 150b), die in Schließstellung gegen die Innenwand der Basis (1; 101) gerichtet ist und deren zumindest ein in der Umgebung jeder Seitenkante (8; 108) liegender Teil in Offenstellung in Berührung mit der Innenwand (3a; 103a) der Basis (1; 101) kommt und somit eines der Mittel zum Begrenzen der Öffnungsbewegung bildet,
wobei die Basis (1; 101) eine im wesentlichen ringförmige Form besitzt und wobei auch sie aufweist:
eine Vorderkante (5a; 105a), die stromauf der Strömung liegt und
eine Hinterkante (5b; 105b), die stromab der Strömung liegt, sowie
eine Außenwand (3b; 103b), die zum Aufnehmen von Nahtmitteln bestimmt ist, wodurch es möglich ist, die Klappe (10; 110) an den Herzgeweben zu befestigen und die Innenwand (3a; 103a), deren Richtung im wesentlichen parallel zu der Strömung liegt und welche die Drehführungs- und Zurückhaltemittel jeder Verschlußklappe (2; 102) trägt,
wobei die Herzklappenprothese (10; 110) dadurch gekennzeichnet ist, daß die Führungs- und Zurückhaltemittel der Verschlußklappen für jede Verschlußklappe (2; 102) aus folgendem bestehen:
zwei Führungsbögen (20; 120) der Hinterkante (7; 107), von denen jeder in der Umgebung einer der Ecken (60; 160) der Verschlußklappe (2; 102) liegt, wobei jeder dieser Bögen (20; 120) auf der Innenwand (3a; 103a) der Basis (1; 101) in einem Punkt seinen Ausgang nimmt, welcher der Hinterkante (5b; 105b) der Basis und dem Übergangsbereich zwischen der Hinterkante der Verschlußklappe und einer der Seitenkanten (8; 108) dieser Verschlußklappe (2; 102) in Offenstellung nahe liegt, wobei sich jeder Bogen (20; 120) gegen den zentralen Teil der Klappe (1; 110) durch einen ersten Teil (16; 116) einer solchen, im wesentlichen kreisförmigen Form erstreckt, daß er in der Umgebung der Hinterkante (7; 107) der Verschlußklappe (2; 102) während deren ganzen Bewegung bleibt, sich den Bogen (20; 120) zu dem Ende stromauf dieses kreisförmigen Teiles (16; 116) nach einem zweiten Teil (17; 117) fortsetzt, welcher die Innenwand (3a; 103a) der Basis (1; 101) in einem Punkt wieder trifft, welcher ihrer Vorderkante (5a; 105a) nahe liegt und als ein Anschlag für die Verschlußklappe (2; 102) in Schließstellung dient und
zumindest einem Führungsbogen (40; 140) der Vorderkante (6; 106), wobei dieser Bogen (40; 140) in einem Punkt der Innenwand (3a, 103a) der Basis (1; 101) seinen Ausgang nimmt, der neben der Vorderkante (6; 106) der Verschlußklappe (2; 102) in Schließstellung von dieser stromauf liegt und eine solche im wesentlichen kreisförmige Form aufweist, daß er in der Umgebung der Vorderkante (6; 106) der Verschlußklappe (2; 102) im Laufe seiner Bewegung bleibt, wobei sich der Führungsbogen (40; 140) der Vorderkante (6; 106) in Richtung des zentralen Teiles der Strömung zumindest bis zu der senkrechten Stellung der Innenfläche (50a; 150a) der Verschlußklappe (2; 102) in Offenstellung fortsetzt, wobei das Ende dieses Bogens einen Anschlag für die Vorderkante (6; 106) der Verschlußklappe (2; 102) in Offenstellung bildet und mit dem stromab liegenden Teil (16; 116) der Führungsbögen (20; 120) der Hinterkante (7; 107) und der Innenwand (3a; 103a) der Basis (1; 101) zusammenarbeitet, um das Entweichen der Verschlußklappe (2; 102) außerhalb ihrer Zurückhaltemittel zu vermeiden, wobei sich das Ende bis in einen Punkt fortsetzt, der sich in Berührung mit der Innenfläche (50a; 150a) der Verschlußklappe (2; 102) in Schließstellung befindet, wodurch ein Stützpunkt der Verschlußklappe (2; 102) in Schließstellung gebildet wird.

2. Herzklappenprothese (10) nach Anspruch 1, dadurch gekennzeichnet, daß sich die Führungsbögen (20) der Hinterkanten (7), welche den Ecken (60) in der Nähe zweier anliegender Verschlußklappen (2) entsprechen, an dem Ende ihres kreisförmigen Teiles (16) wieder treffen und den zweiten Teil (17) gemeinsam aufweisen, welcher die Innenwand (3a) der Basis (1) wieder trifft und gleichzeitig als Anschlag für die zwei Verschlußklappen (2) in Schließstellung dient.

3. Herzklappenprothese (110) nach Anspruch 1, dadurch gekennzeichnet, daß die Führungsbögen (120) der Hinterkante (107) jeder Verschlußklappe (102) aus den Kanten einer Senke (115) bestehen, die in einem Höcker (130) der Innenwand (103a) der Basis (101) ausgehöhlt ist.

4. Herzklappenprothese (110) nach Anspruch 3, dadurch gekennzeichnet, daß jeder Höcker (130) der Innenwand (103a) der Basis (101) zwei Senken (115) aufnimmt, welche den Führungsbögen (120) der Hinterkanten (107) zweier anliegender Verschlußklappen (102) entsprechen, wobei jede dieser Senken (115) an einer der zwei Flächen des Höckers (130) ausgehöhlt ist.

5. Herzklappenprothese (10; 110) nach irgendeinem der Ansprüche 1 bis 4, dadurch gekennzeichnet, daß jeder der Führungsbögen (40; 140) der Vorderkante (6; 106) jeder Verschlußklappe (2; 102) in der Nähe eines Punktes der Vorderkante jeder Verschlußklappe liegt, der sich im Laufe der Bewegung der Verschlußklappe am wenigsten verschiebt.

6. Herzklappenprothese (10; 110) nach irgendeinem der Ansprüche 1 bis 5, dadurch gekennzeichnet, daß die Vorderkante (5a; 105a) der Basis (1; 101) auf der ganzen oder einem Teil ihrer Länge einen gegen das Zentrum der Strömung ausgerichteten Wulst aufweist, der sich mit der Vorderkante der Verschlußklappen (2; 102) in Schließstellung in Berührung befindet und es somit ermöglicht, deren Dichtigkeit in der Schließstellung zu verbessern.

7. Herzklappenprothese (10; 110) nach irgendeinem der Ansprüche 1 bis 6, dadurch gekennzeichnet, daß die Basis (1; 101) einen im wesentlichen kreisförmigen Abschnitt aufweist und daß die Anzahl der Verschlußklappen (2, 102) drei beträgt.

8. Herzklappenprothese nach irgendeinem der Ansprüche 1 bis 6, dadurch gekennzeichnet, daß die Basis einen im wesentlichen elliptischen Abschnitt aufweist und daß die Anzahl der Verschlußklappen zwei beträgt, wobei sich ihre Hinterkanten in einer geschlossenen Stellung längs einer Linie in der Nähe der größten Achse der Ellipse wieder treffen.

9. Herzklappenprothese (10) nach irgendeinem der Ansprüche 1 bis 8, dadurch gekennzeichnet, daß sie zumindest ein zusätzliches Führungsmittel für jede Verschlußklappe (2) aufweist, wobei das zusätzliche Führungsmittel für jede Verschlußklappe (2) aus zumindest einem Höcker (oder einer Verformung 70) der Innenwand (3a) der Basis (1) besteht, der sich in einem Punkt in der Nähe der Vorderkante (6) der Verschlußklappe (2) in Schließstellung und von dieser stromab befindet und derart angeordnet ist, daß sein Ende im Laufe der Bewegung in der Umgebung der Außenfläche (50b) der Verschlußklappe (2) bleibt und somit als eine Stütze für die Verschlußklappe (2) im Laufe ihres Schwenkens dient.

10. Herzklappenprothese nach irgendeinem der Ansprüche 1 bis 9, dadurch gekennzeichnet, daß jede der Verschlußklappen (2; 102) mit Vortreibmitteln zum Verschließen versehen ist, nämlich mit Mitteln, die zum Vorwegnehmen des Verschließens der Verschlußklappen bei Rückkehr der Blutströmung in Gegenrichtung bestimmt sind.

11. Herzklappenprothese nach Anspruch 10, dadurch gekennzeichnet, daß die Vortreibmittel zum Verschließen aus magnetischen Massen entgegengesetzter Polungen bestehen, die auf der Höhe der Ecken jeder Verschlußklappe (2; 102) angebracht sind, wobei die magnetischen Polungen der Ecken der zwei angrenzenden Verschlußklappen entgegengesetzt sind.

12. Herzklappenprothese nach Anspruch 11, dadurch gekennzeichnet, daß die auf der Höhe der Ecken (60, 160) jeder Verschlußklappe (2; 102) angebrachten magnetischen Massen mit zusätzlichen magnetischen Massen zusammenwirken, die in der Basis (1; 101) zwischen zwei angrenzenden Verschlußklappen angeordnet sind und deren Abmessung im wesentlichen gleich dem Abstand ist, der zwischen den anliegenden Ecken zweier angrenzender Verschlußklappen in Offenstellung vorhanden ist, wobei jede zusätzliche magnetische Masse in der Basis so angeordnet ist, daß ihre Polung den Polungen entgegengesetzten Vorzeichens der magnetischen Massen entsprechen, die in den Ecken der Verschlußklappen angebracht sind.

13. Herzklappenprothese nach irgendeinem der Ansprüche 11 und 12, dadurch gekennzeichnet, daß die magnetischen Massen aus kleinen Magnenten bestehen.

14. Herzklappenprothese nach irgendeinem der Ansprüche 11 und 12, dadurch gekennzeichnet, daß die magnetischen Massen aus einem magnetischen Material bestehen, das bei der Herstellung jeder Verschlußklappe (2; 102) oder der Basis (1; 101) eingearbeitet wird.
